(19) Europäisches Patentamt
European Patent Office
Office européen des brevets

(11) EP 1 704 408 B1

(12) # FASCICULE DE BREVET EUROPEEN

(45) Date de publication et mention de la délivrance du brevet:
**20.10.2010 Bulletin 2010/42**

(21) Numéro de dépôt: **04805877.0**

(22) Date de dépôt: **03.12.2004**

(51) Int Cl.:
***G01N 27/12*** (2006.01) ***G01N 29/02*** (2006.01)
***G01N 33/00*** (2006.01) ***C08L 83/16*** (2006.01)

(86) Numéro de dépôt international:
**PCT/FR2004/050646**

(87) Numéro de publication internationale:
**WO 2005/057198 (23.06.2005 Gazette 2005/25)**

(54) **UTILISATION DE POLYMERES OU DE COMPOSITES A BASE DE SILOXANES DANS DES CAPTEURS CHIMIQUES POUR LA DETECTION DE COMPOSES NITRES.**

VERWENDUNG VON POLYMEREN ODER VERBUNDSTOFFEN AUF SILOXANBASIS IN CHEMISCHEN SENSOREN ZUM NACHWEIS VON NITRATVERBINDUNGEN

USE OF SILOXANE-BASED POLYMERS OR COMPOSITES IN CHEMICAL SENSORS FOR DETECTING NITRATE COMPOUNDS

(84) Etats contractants désignés:
**AT BE BG CH CY CZ DE DK EE ES FI FR GB GR HU IE IS IT LI LT LU MC NL PL PT RO SE SI SK TR**

(30) Priorité: **05.12.2003 FR 0350984**

(43) Date de publication de la demande:
**27.09.2006 Bulletin 2006/39**

(73) Titulaire: **Commissariat à l'Énergie Atomique et aux Énergies Alternatives**
**75015 Paris (FR)**

(72) Inventeurs:
- **LEBRET, Bruno**
  **F-37300 Joue-Les-Tours (FR)**
- **HAIRAULT, Lionel**
  **F-37150 BLERE (FR)**
- **PASQUINET, Eric**
  **F-37550 Saint Avertin (FR)**

(74) Mandataire: **Poulin, Gérard et al**
**BREVALEX**
**3, rue du Docteur Lancereaux**
**75008 Paris (FR)**

(56) Documents cités:
**WO-A-01/77664** **WO-A-02/08234**
**WO-A-02/23134** **WO-A-98/22795**
**DE-C- 19 708 529** **FR-A- 2 815 351**
**US-A1- 2003 168 355** **US-B1- 6 433 694**

- **MCGILL R A ET AL: "The design of functionalized silicone polymers for chemical sensor detection of nitroaromatic compounds" SENSORS AND ACTUATORS B, ELSEVIER SEQUOIA S.A., LAUSANNE, CH, vol. 65, no. 1-3, 30 juin 2000 (2000-06-30), pages 5-9, XP004208582 ISSN: 0925-4005**
- **ALI M B ET AL: "Sensitive cyclodextrin-polysiloxane gel membrane on EIS structure and ISFET for heavy metal ion detection" SENSORS AND ACTUATORS B, ELSEVIER SEQUOIA S.A., LAUSANNE, CH, vol. 62, no. 3, mars 2000 (2000-03), pages 233-237, XP004194220 ISSN: 0925-4005**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 704 408 B1

## Description

La présente invention se rapporte à l'utilisation de polymères à base de siloxanes ou de composites comprenant un tel polymère et une ou plusieurs charges conductrices de l'électricité en tant que matériaux sensibles dans des capteurs destinés à détecter des composés nitrés, et en particulier des composés nitroaromatiques tels que le nitrobenzène (NB), le dinitrobenzène (DNB), le trinitrobenzène (TNB), le nitrotoluène (NT), le dinitrotoluène (DNT), le 2,4,6-trinitrotoluène (TNT) et analogues.

De tels capteurs sont utiles pour la détection d'explosifs, que ce soit en vue d'assurer la sécurité de lieux publics comme les aéroports, de contrôler la licéité de marchandises en circulation sur un territoire, de lutter contre le terrorisme, de procéder à des opérations de désarmement, de localiser des mines antipersonnel ou encore de dépolluer des sites industriels ou militaires.

Ils sont également utiles pour la protection de l'environnement, en particulier pour le contrôle et la surveillance de la pollution atmosphérique et de la qualité d'ambiances plus ou moins confinées, ainsi que pour la surveillance à des fins sécuritaires, de sites industriels fabriquant, stockant et/ou manipulant des composés nitrés.

## ETAT DE LA TECHNIQUE ANTERIEURE

La détection d'explosifs est un problème d'intérêt crucial, notamment en matière de sécurité civile.

A l'heure actuelle, plusieurs méthodes sont utilisées pour détecter des vapeurs de composés nitrés entrant dans la constitution des explosifs, comme l'emploi de chiens "*renifleurs*" dressés et entraînés à cet effet, l'analyse en laboratoire, par exemple par chromatographie couplée à un spectromètre de masse ou à un détecteur à capture d'électrons, d'échantillons prélevés sur site, ou encore la détection infrarouge.

Ces méthodes font, d'une manière générale, preuve d'une grande sensibilité, ce qui est primordial en matière de détection d'explosifs compte tenu de la très faible concentration en vapeurs de composés nitrés qui règne au voisinage d'un explosif. Elles ne donnent toutefois pas totalement satisfaction.

Ainsi, l'utilisation de chiens "*renifleurs*" présente l'inconvénient de nécessiter une longue formation des chiens et de leurs maîtres et d'être inadaptée à des opérations prolongées en raison de ce que la durée d'attention des chiens est limitée.

Quant aux autres méthodes, l'encombrement des appareillages qu'elles utilisent, leur consommation d'énergie et leurs coûts de mise en oeuvre s'opposent au développement de systèmes de détection aisément transportables et autonomes et, partant, aptes à être utilisés sur tout type de sites.

Depuis quelques années, le développement de capteurs capables de détecter en temps réel des espèces chimiques gazeuses est en plein essor. Le fonctionnement de ces capteurs est basé sur l'utilisation d'un film d'un matériau sensible, c'est-à-dire d'un matériau dont au moins une propriété physique est modifiée au contact des molécules gazeuses recherchées, qui revêt un système apte à mesurer en temps réel toute variation de cette propriété physique et de mettre ainsi en évidence la présence des molécules gazeuses recherchées.

Les avantages des capteurs chimiques par rapport aux méthodes précitées sont multiples : instantanéité des résultats, possibilité de miniaturisation et, donc, portabilité, maniabilité et autonomie importante, faibles coûts de fabrication et d'exploitation, etc.

Toutefois, il est évident que leurs performances sont extrêmement variables selon la nature du matériau sensible utilisé.

Pour la détection de composés nitrés gazeux, et plus particulièrement de composés nitroaromatiques, de nombreux matériaux sensibles ont déjà été proposés parmi lesquels on peut citer le silicium poreux, le charbon végétal, le polyéthylène glycol, les amines, les cyclodextrines, des cavitands et des polymères fluorescents (références **[1]** à **[5])**.

Par ailleurs, l'utilisation potentielle de polysiloxanes fonctionnalisés en tant que matériaux sensibles de capteurs destinés à détecter des composés nitroaromatiques a été étudiée par McGill *et al.* (référence **[6]**).

Ces Auteurs se sont attachés à déterminer les paramètres de solubilité de quelques composés nitroaromatiques (NB, NT, TNB, DNT, TNT) et à définir, à partir de ces paramètres, leurs propriétés de sorption à l'état de vapeurs (c'est-à-dire leur aptitude à être absorbés et retenus) dans une série de polymères incluant divers polysiloxanes.

McGill et *al*., XP 4208582, déduisent des résultats qu'ils obtiennent que les composés nitroaromatiques sont susceptibles d'interagir avec des polymères d'autant plus fortement que ces polymères présentent des propriétés de solubilité complémentaires aux leurs. Ils en concluent que les polysiloxanes les plus prometteurs pour la détection de composés nitroaromatiques sont ceux dont les monomères comportent un cycle aromatique porteur d'un ou plusieurs groupes aptes à établir des liaisons hydrogène avec ces composés, par exemple un groupe hexafluoroisopropanol (HFIP). Il est de fait que les essais de détection du DNT qu'ils effectuent au moyen d'un capteur à ondes de surface muni d'un film mince d'un polysiloxane issu de monomères à cycle aromatique porteur d'un groupe pendant HFIP apparaissent donner des résultats satisfaisants.

Or, dans le cadre de leurs travaux sur le développement de capteurs destinés plus spécialement à détecter

des explosifs, les Inventeurs ont constaté que, de manière tout à fait surprenante, des capteurs utilisant comme matériaux sensibles, des polymères à base de siloxanes qui ne comportent ni cycle aromatique, ni groupe pendant de type HFIP, détectent les composés nitrés, et en particulier les composés nitroaromatiques, avec une sensibilité nettement plus élevée que des capteurs utilisant les polysiloxanes préconisés par McGill et *al.*.

C'est cette constatation qui est à la base de l'invention.

## EXPOSÉ DE L'INVENTION

L'invention a pour objet l'utilisation d'au moins un polymère comprenant au moins un motif répétitif siloxane répondant à la formule générale (I) ci-après :

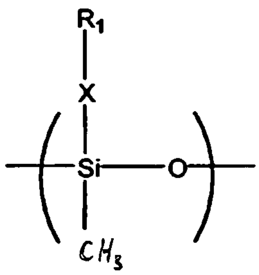

(I)

dans laquelle :

X un groupe hydrocarboné linéaire, saturé ou insaturé, et comprenant de 1 à 50 atomes de carbone ; tandis que $R_1$ représente un groupe CN, un groupe $C(Z)_3$, $CH(Z)_2$ ou $CH_2Z$ avec Z représentant un atome d'halogène ;

ou d'un composite comprenant ce polymère et une ou plusieurs charges conductrices de l'électricité, en tant que matériau sensible dans un capteur destiné à détecter un ou plusieurs composés nitrés.

Parmi les motifs répétitifs siloxane de formule particulière (Id), on préfère notamment ceux dans lesquels X représente une chaîne alkylène comportant de 2 à 10 atomes de carbone (c'est-à-dire une chaîne $(CH_2)_n$ dans laquelle n va de 2 à 10) et, parmi ces derniers, le trifluoropropylméthylsiloxane ($X = (CH_2)_2$, $R_1 = CF_3$) et le cyanopropylméthylsiloxane ($X = (CH_2)_3$, $R_1$ = CN).

Selon une autre disposition préférée de l'invention, le polymère est un homopolymère, c'est-à-dire qu'il n'est constitué que d'un seul et même motif répétitif siloxane de formule générale (I), auquel cas, il est avantageusement choisi parmi les polytrifluoropropylméthylsiloxanes et les polycyanopropylméthylsiloxanes, et plus particulièrement parmi ceux qui présentent un poids moléculaire moyen allant de 50 à 100 000.

En variante, le polymère peut également être un copolymère, auquel cas il peut aussi bien être constitué de différents motifs répétitifs siloxane répondant tous à la formule générale (I) que comprendre un ou plusieurs motifs répétitifs siloxane de formule générale (I) et un ou plusieurs motifs répétitifs autres, siloxane ou non.

En effet, il peut, par exemple, être utile d'inclure dans le polymère des motifs répétitifs issus d'un monomère du type éthylène, propylène, oxyde d'éthylène, styrène, carbazole de vinyle ou encore acétate de vinyle, aptes à lui conférer une meilleure résistance mécanique dans le cas notamment où l'on souhaite l'utiliser sous la forme d'un film mince.

Les polymères présentant un motif répétitif siloxane de formule générale (I) n'étant pas des conducteurs intrinsèques de l'électricité, il est possible, conformément à l'invention, de les mélanger à une ou plusieurs charges conductrices en quantité suffisante pour que les composites résultants aient une conductivité électrique adaptée à leur utilisation comme matériaux sensibles de capteurs résistifs. Ces charges conductrices peuvent être, par exemple, des particules de noir de carbone ou des poudres de métaux (Cu, Pd, Au, Pt, ...) ou d'oxydes métalliques ($V_2O_3$, TiO, ...).

Selon encore une autre disposition préférée de l'invention, le polymère ou le composite se présente sous la forme d'un film mince qui recouvre l'une ou les deux faces d'un substrat convenablement choisi en fonction de la propriété physique du matériau sensible dont les variations sont destinées à être mesurées par ce capteur.

En variante, le polymère ou le composite peut également se présenter sous une forme massive comme, par exemple, un cylindre présentant une certaine porosité de sorte à rendre accessible aux composés nitrés l'ensemble des molécules formant ledit polymère ou ledit composite.

Lorsqu'il se présente sous la forme d'un film mince, ce dernier présente, de préférence, une épaisseur de 10

angströms à 100 microns.

Un tel film peut être obtenu par l'une quelconque des techniques proposées à ce jour pour réaliser un film mince sur la surface d'un substrat, par exemple :

- par pulvérisation, par dépôt à la tournette ("*spin coating*" en langue anglo-saxonne) ou par dépôt-évaporation ("*drop coating*" en langue anglo-saxonne) sur le substrat d'une solution contenant le polymère ou le composite,
- par trempage-retrait ("*dip coating*" en langue anglo-saxonne) du substrat dans une solution contenant le polymère ou le composite,
- par la technique de Langmuir-Blodgett,
- par dépôt électrochimique, ou encore
- par polymérisation *in situ*, c'est-à-dire directement sur la surface du substrat, d'un monomère précurseur du polymère.

Le substrat ainsi que le système de mesure du capteur sont choisis en fonction de la propriété physique du polymère ou du composite dont les variations induites par la présence de composés nitrés sont destinées à être mesurées par le capteur.

En l'espèce, les variations de deux propriétés physiques se sont révélées particulièrement intéressantes à mesurer : les variations de masse dans le cas d'un polymère et les variations de conductivité électrique dans le cas d'un composite.

Aussi, le capteur est-il, de préférence, un capteur gravimétrique pour la mesure de variations de masse, ou un capteur résistif pour la mesure de variations de conductivité électrique.

A titre d'exemples de capteurs gravimétriques, on peut citer les capteurs du type à microbalance à quartz, les capteurs à ondes de surface, plus connus sous la terminologie anglo-saxonne "SAW" pour "Surface Acoustic Wave", tels que les capteurs à ondes de Love et les capteurs à ondes de Lamb, ainsi que les microleviers.

Parmi les capteurs gravimétriques, on préfère plus particulièrement les capteurs du type microbalance à quartz. Ce type de capteurs, dont le principe de fonctionnement est décrit dans la référence **[2],** comprend, schématiquement, un substrat piézo-électrique (ou résonateur), généralement un cristal de quartz recouvert sur ses deux faces d'une couche métallique, par exemple d'or ou de platine, et qui est relié à deux électrodes. Le matériau sensible recouvrant l'une ou les deux faces du substrat, toute variation de masse de ce matériau se traduit par une variation de la fréquence de vibration du substrat.

Bien entendu, il est également possible d'utiliser un polymère ou un composite tel que précédemment défini, comme matériau sensible dans des capteurs conçus pour mesurer des variations d'une propriété physique autre que la masse et la conductivité électrique comme, par exemple, des variations d'une propriété optique telles que de fluorescence, de luminescence, d'absorbance dans le domaine UV-visible ou de longueur d'onde dans le domaine des infrarouges.

Dans ce cas, il est possible soit d'exploiter une propriété optique intrinsèque du polymère ou du composite dans le cas où celui-ci en possède une (absorbance, spectre IR, ...), soit de conférer à ce polymère ou à ce composite une propriété optique particulière par couplage avec un marqueur approprié, par exemple fluorescent ou luminescent.

Par ailleurs, il est également possible de réunir au sein d'un même dispositif ou "*multicapteur*", plusieurs capteurs comprenant des matériaux sensibles différents les uns des autres, ou munis de substrats et de systèmes de mesure différents les uns des autres comme, par exemple, un ou plusieurs capteurs gravimétriques et/ou un ou plusieurs capteurs résistifs, l'essentiel étant que l'un au moins de ces capteurs comprenne un polymère ou un composite tel que précédemment défini.

Selon encore une disposition préférée de l'invention, le ou les composés nitrés destinés à être détectés par le capteur sont choisis parmi les composés nitroaromatiques, les nitramines, les nitrosamines et les esters nitriques, ces composés pouvant aussi bien se présenter sous forme solide, liquide ou gazeuse (vapeurs).

A titre d'exemples de composés nitroaromatiques, on peut citer le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'amino-dinitrotoluène, le dinitrotrifluorométhylbenzène, le chlorodinitrotrifluorométhylbenzène, l'hexanitrostilbène, la trinitrophénylméthylnitramine (ou tétryle) ou encore le trinitrophénol (ou acide picrique).

Les nitramines sont, elles, par exemple la cyclotétraméthylènetétranitramine (ou octogène), la cyclotriméthylènetrinitramine (ou hexogène) et le tétryl, tandis que les nitrosamines sont, par exemple, la nitrosodiméthylamine.

Quant aux esters nitriques, il s'agit, par exemple, de pentrite, de dinitrate d'éthylène glycol, de dinitrate de diéthylène glycol, de nitroglycérine ou de nitroguanidine.

Des capteurs comportant un polymère ou un composite tel que précédemment défini, comme matériau sensible, se sont révélés présenter de nombreux avantages, notamment :

- une aptitude à détecter les composés nitrés, et en particulier les composés nitroaromatiques, avec une très grande sensibilité puisqu'ils sont capables de détecter leur présence à des concentrations de l'ordre du ppm (partie par

million), voire du dixième de ppm,

- une rapidité de réponse et une reproductibilité de cette réponse,
- une aptitude à fonctionner en continu,
- une stabilité des performances dans le temps,
- une durée de vie très satisfaisante,
- un coût de fabrication compatible avec une production de capteurs en série, une très faible quantité de polymère ou de composite (c'est-à-dire en pratique de quelques mg) étant nécessaire pour la fabrication d'un capteur, et
- la possibilité d'être miniaturisés et, partant, d'être aisément transportables et manipulables sur tout type de sites.

Ils sont donc particulièrement utiles pour détecter des explosifs, notamment dans des lieux publics.

D'autres caractéristiques et avantages de l'invention apparaîtront mieux à la lecture du complément de description qui suit, qui se rapporte à des exemples d'utilisation de films minces de polytrifluoropropylméthylsiloxane et de polycyanopropylméthylsiloxane dans des capteurs à microbalance à quartz pour la détection de vapeurs de dinitrotrifluorométhoxybenzène (DNTFMB) et de dinitrobenzène (DNB), et qui se réfère aux dessins annexés.

Le choix du DNTFMB et du DNB en tant que composés nitrés à détecter, a été motivé par le fait que ces composés sont très proches du dinitrotoluène (DNT), lequel est le dérivé nitré le plus présent dans la signature chimique des mines à base de trinitrotoluène (TNT).

Bien entendu, les exemples qui suivent ne sont donnés qu'à titre d'illustrations de l'objet de l'invention et ne constituent en aucun cas une limitation de cet objet.

## BREVE DESCRIPTION DES DESSINS

La figure 1 représente l'évolution de la fréquence de vibration (courbe A) du quartz d'un capteur à microbalance à quartz comprenant un film mince de polytrifluoropropylméthylsiloxane au cours de deux cycles d'exposition (courbe B) de ce capteur à des vapeurs de DNTFMB de concentration égale à 3 ppm.

La figure 2 représente l'évolution de la fréquence de vibration (courbe A) du quartz d'un capteur à microbalance à quartz comprenant un film mince de polytrifluoropropylméthylsiloxane au cours de deux cycles d'exposition (courbe B) de ce capteur à des vapeurs de DNB de concentration égale à 150 ppb (partie par billion).

La figure 3 représente l'évolution de la fréquence de vibration (courbe A) du quartz d'un capteur à microbalance à quartz comprenant un film mince de polycyanopropylméthylsiloxane au cours d'un cycle d'exposition (courbe B) de ce capteur à des vapeurs de DNTFMB de concentration égale à 3 ppm.

La figure 4 représente l'évolution de la fréquence de vibration (courbe A) du quartz d'un capteur à microbalance à quartz comprenant un film mince de polycyanopropylméthylsiloxane au cours de deux cycles d'exposition (courbe B) de ce capteur à des vapeurs de DNTFMB de concentration égale à 1 ppm pour le premier cycle et à 0,1 ppm pour le deuxième.

La figure 5 représente les valeurs des variations de la fréquence de vibration (ΔF) du quartz d'un capteur à microbalance à quartz comprenant un film mince de polycyanopropylméthylsiloxane telles qu'obtenues en soumettant ce capteur à douze expositions à des vapeurs de DNTFMB de 10 minutes chacune, sur une période de 150 jours.

## EXEMPLES

### Exemple 1 : détection du DNTFMB par un capteur comprenant un film mince de polytrifluoropropylméthylsiloxane

Dans cet exemple, on utilise un capteur à microbalance à quartz comprenant un quartz de coupe AT, de fréquence de vibration de 9 MHz, recouvert de deux électrodes de mesure circulaires en or (modèle QA9RA-50, AMETEK PRECISION INSTRUMENTS) et portant sur ses deux faces un film mince de polytrifluoropropylméthylsiloxane.

Le dépôt de ce film est réalisé en effectuant sur chaque face du quartz 6 pulvérisations de 0,5 secondes chacune d'une solution de polytrifluoropropylméthylsiloxane (société ABCR, référence FMS-9921) dans du chloroforme, de concentration égale à 5 g/l.

La variation de la fréquence de vibration du quartz due à ce dépôt est de 8,1 kHz.

Le capteur est soumis à deux cycles d'exposition à des vapeurs de DNTFMB, à température ambiante :

- le premier cycle comprenant une phase d'exposition de 5800 secondes à l'air ambiant, suivie d'une phase d'exposition de 600 secondes aux vapeurs de DNTFMB, puis d'une phase d'exposition de 2600 secondes à l'air ambiant ;

- le deuxième cycle comprenant une phase d'exposition de 600 secondes aux vapeurs de DNTFMB, suivie d'une phase d'exposition de 4800 secondes à l'air ambiant ;

la concentration du DNTFMB étant de 3 ppm dans les deux cycles.

La figure 1 montre l'évolution de la fréquence de vibration du quartz au cours de ces deux cycles, la courbe A et la courbe B correspondant aux variations respectives de ladite fréquence (F), exprimée en hertz (Hz), et de la concentration en DNTFMB ([C]), exprimée en ppm, en fonction du temps (t), exprimé en secondes.

**Exemple 2 : détection du DNB par un capteur comprenant un film mince de polytrifluoropropylméthylsiloxane**

Dans cet exemple, on utilise un capteur à microbalance à quartz comprenant un quartz identique à celui utilisé dans l'exemple 1, mais dans lequel le quartz est recouvert sur ses deux faces d'un film mince de polytrifluoropropylméthysiloxane d'épaisseur légèrement supérieure à celui utilisé dans l'exemple 1.

Le dépôt de ce film est réalisé en effectuant sur chaque face du quartz 19 pulvérisations de 0,2 secondes chacune d'une solution de polytrifluoropropylméthylsiloxane dans du chloroforme, de concentration égale à 2 g/l.

La variation de la fréquence de vibration du quartz due à ce dépôt est de 9,9 kHz.

Le capteur est soumis à deux cycles d'exposition à des vapeurs de DNB, à température ambiante :

- le premier cycle comprenant une phase d'exposition de 1700 secondes à l'air ambiant, suivie d'une phase d'exposition de 600 secondes aux vapeurs de DNB, puis d'une phase d'exposition de 2300 secondes à l'air ambiant ;

- le deuxième cycle comprenant une phase d'exposition de 600 secondes aux vapeurs de DNB, suivie d'une phase d'exposition de 1800 secondes à l'air ambiant ;

la concentration du DNB étant de 150 ppb dans les deux cycles.

La figure 2 montre l'évolution de la fréquence de vibration du quartz au cours de ces deux cycles, la courbe A et la courbe B correspondant aux variations respectives de ladite fréquence (F), exprimée en Hz, et de la concentration en DNB ([C]), exprimée en ppb, en fonction du temps (t), exprimé en secondes.

**Exemple 3 : détection du DNTFMB par un capteur comprenant un film mince de polycyanopropylméthylsiloxane**

Dans cet exemple, on utilise un capteur à microbalance à quartz comprenant un quartz identique à celui utilisé dans l'exemple 1, mais dans lequel le quartz est recouvert sur ses deux faces d'un film mince de polycyanopropylméthylsiloxane.

Le dépôt de ce film est réalisé en effectuant sur chaque face du quartz 12 pulvérisations de 0,2 secondes chacune d'une solution de polycyanopropylméthylsiloxane (société ABCR, référence YMS-T31) dans du chloroforme, de concentration égale à 5 g/l.

La variation de la fréquence de vibration du quartz due à ce dépôt est de 8,5 kHz.

Le capteur est soumis à un cycle d'exposition à des vapeurs de DNTFMB de concentration égale à 3 ppm, à température ambiante, ce cycle comprenant une phase d'exposition de 3000 secondes à l'air ambiant, suivie d'une phase d'exposition de 600 secondes aux vapeurs de DNTFMB, puis d'une phase d'exposition de 11400 secondes à l'air ambiant.

La figure 3 montre l'évolution de la fréquence de vibration du quartz au cours de ce cycle, la courbe A et la courbe B correspondant aux variations respectives de ladite fréquence (F), exprimée en Hz, et de la concentration en DNTFMB ([C]), exprimée en ppm, en fonction du temps (t), exprimé en secondes.

**Exemple 4 : détection du DNTFMB par un capteur comprenant un film mince de polycyanopropylméthylsiloxane**

Dans cet exemple, on utilise un capteur à microbalance à quartz comprenant un quartz identique à celui utilisé dans l'exemple 1, mais dans lequel le quartz est recouvert sur ses deux faces d'un film mince de polycyanopropylméthylsiloxane.

Le dépôt de ce film est réalisé en effectuant sur chaque face du quartz deux pulvérisations de 0,5 secondes chacune d'une solution de polycyanopropylméthylsiloxane (société ABCR, référence YMS-T31) dans du chloroforme, de concentration égale à 5 g/l.

La variation de la fréquence de vibration du quartz due à ce dépôt est de 2 kHz.

Le capteur est soumis à deux cycles d'exposition à du DNTFMB sous forme de vapeurs, à température ambiante :

- le premier cycle comprenant une phase d'exposition de 1300 secondes à l'air ambiant, suivie d'une phase d'exposition de 600 secondes au DNTFMB à une concentration de 1 ppm, puis d'une phase d'exposition de 6400 secondes à l'air ambiant ;
- le deuxième cycle comprenant une phase d'exposition de 600 secondes au DNTFMB à une concentration de 0,1 ppm, suivie d'une phase d'exposition de 1100 secondes à l'air ambiant.

La figure 4 montre l'évolution de la fréquence de vibration du quartz au cours de ces deux cycles, la courbe A et la courbe B correspondant aux variations respectives de ladite fréquence (F), exprimée en hertzs (Hz), et de la concentration en DNTFMB ([C]), exprimée en ppm, en fonction du temps (t), exprimé en secondes.

**Exemple 5 : étude de la stabilité dans le temps des performances d'un capteur comprenant un film mince de polycyanopropylméthylsiloxane**

Dans cet exemple, on utilise un capteur à microbalance à quartz identique à celui utilisé dans l'exemple 4.
Ce capteur est soumis à une première exposition à des vapeurs de DNTFMB de concentration égale à 3 ppm, à température ambiante et pendant 10 minutes, puis il est conservé à l'air ambiant.
Il est ensuite soumis à onze autres expositions à des vapeurs de DNTFMB de concentration égale à 3 ppm, toujours à température ambiante et d'une durée de 10 minutes chacune, réparties sur une période de 150 jours.
La figure 5 représente les valeurs des variations de la fréquence de vibration ($\Delta F$) du quartz observées au cours de ces douze expositions, ces valeurs étant déterminées pour chaque exposition comme suit :

$$\Delta F = \text{fréquence de vibration au temps } t_0 \text{ de l'exposition} - \text{fréquence de vibration au temps } t_{10min} \text{ de l'exposition,}$$

et symbolisées par des losanges sur ladite figure 5.

**Exemple 6 : comparaison des performances d'un capteur comprenant un film mince d'un polysiloxane utile selon l'invention et d'un capteur comprenant un film mince d'un polysiloxane préconisé par McGill et *al.***

Dans cet exemple, on utilise deux capteurs à microbalance à quartz comprenant tous deux un quartz identique à celui utilisé dans l'exemple 1, mais se différenciant l'un de l'autre en ce que le quartz du premier est recouvert sur ses deux faces d'un film mince de polycyanopropylméthylsiloxane, alors que le quartz du second est recouvert d'un film mince d'un polysiloxane dont les monomères comportent un cycle aromatique et deux groupes pendants HFIP.
Ce polysiloxane répond à la formule (II) ci-après :

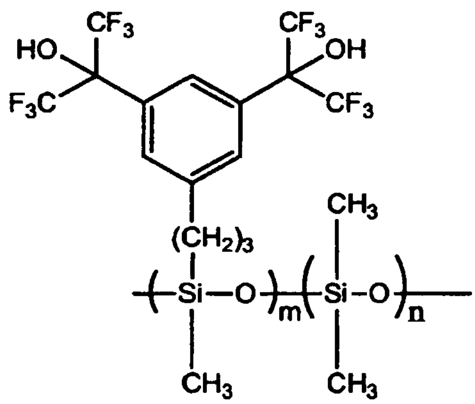

m = 47, n = 53

Les dépôts des films sont réalisés de sorte que la variation de la fréquence de vibration des quartz due à ces

dépôts soit égale à 2 kHz pour chacun des capteurs.

Pour ce faire, le dépôt du film de polycyanopropylméthylsiloxane est effectué comme décrit dans l'exemple 4, tandis que le dépôt du film du polysiloxane de formule (II) est effectué en pulvérisant 6 fois 0,2 secondes une solution dudit polysiloxane dans du dichlorométhane, de concentration égale à 2 g/l, sur les deux faces du quartz.

Les deux capteurs sont exposés, exactement dans les mêmes conditions, à des vapeurs de DNTFMB de concentration égale à 3 ppm, à température ambiante et pendant 10 minutes.

La mesure de la fréquence de vibration du quartz des deux capteurs au temps $t_0$ et au temps $t_{10min}$ de cette exposition donne une variation de la fréquence de vibration de 600 Hz pour le quartz du capteur comprenant le film mince de polycyanopropylméthylsiloxane, et de 200 Hz - soit 3 fois plus faible - pour le quartz du capteur comprenant le film mince du polysiloxane de formule (II).

Les exemples 1 à 4 ci-avant montrent que des capteurs comprenant un matériau sensible conforme à l'invention, sont capables de détecter avec une très grande sensibilité des composés nitrés comme le DNTFMB et le DNB. Ils montrent aussi que la réponse de ces capteurs est à la fois réversible et reproductible.

L'exemple 5 montre de plus que les performances de ces capteurs sont stables dans le temps et qu'ils sont toujours capables, cinq mois après leur élaboration, de détecter de très faibles quantités de DNTFMB.

Enfin, l'exemple 6 montre que ces capteurs présentent, à l'égard des composés nitroaromatiques, une sensibilité très nettement supérieure à celle d'un capteur comprenant un film mince d'un polysiloxane tel que préconisé par McGill et *al.*.

## REFERENCES CITEES

**[1]** Content et al., Chem. Eur. J., 6, 2205, 2000

**[2]** Sanchez-Pedrono et al., Anal. Chim. Acta, 182, 285, 1986

**[3]** Yang et al., Langmuir, 14, 1505, 1998

**[4]** Nelli et al., Sens. Actuators B, 13-14, 302, 1993

**[5]** Yang et al., J. Am. Chem. Soc., 120, 11864, 1998

**[6]** McGill et al., Sensors and Actuators B65, 5-9, 2000


## Revendications

1. Utilisation d'au moins un polymère comprenant au moins un motif répétitif siloxane répondant à la formule générale (I) ci-après :

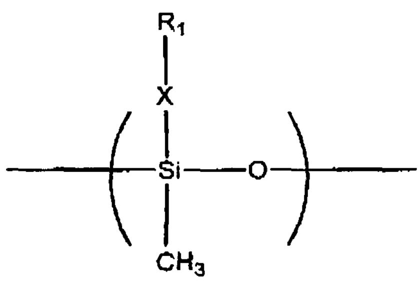

(I)

dans laquelle X est un groupe hydrocarboné linéaire, saturé ou insaturé, et comportant de 1 à 50 atomes de carbone, tandis que $R_1$ représente
un groupe CN, un groupe $C(Z)_3$, $CH(Z)_2$ ou $CH_2Z$ avec Z représentant un atome d'halogène ;
ou d'un composite comprenant ce polymère et une ou plusieurs charges conductrices de l'électricité, en tant que matériau sensible dans un capteur destiné à détecter un ou plusieurs composés nitrés.

2. Utilisation selon la revendication 1, dans laquelle, dans la formule (I), X représente une chaîne alkylène comportant de 2 à 10 atomes de carbone.

3. Utilisation selon la revendication 1 ou la revendication 2, dans laquelle le motif répétitif siloxane est le trifluoropropylméthylsiloxane ou le cyanopropylméthylsiloxane.

4. Utilisation selon l'une quelconque des revendications précédentes, dans laquelle le polymère est choisi parmi les polytrifluoropropylméthylsiloxanes et les polycyanopropylméthylsiloxanes.

5. Utilisation selon la revendication 4, dans laquelle le polymère a un poids moléculaire moyen allant de 50 à 100 000.

6. Utilisation selon l'une quelconque des revendications précédentes, dans laquelle la ou les charges conductrices du composite sont choisies parmi les particules de noir de carbone et les poudres de métaux et d'oxydes métalliques.

7. Utilisation selon l'une quelconque des revendications précédentes, dans laquelle le polymère ou le composite est utilisé sous la forme d'un film mince recouvrant l'une ou les deux faces d'un substrat.

8. Utilisation selon la revendication 7, dans laquelle le film mince mesure de 10 angströms à 100 microns d'épaisseur.

9. Utilisation selon la revendication 7 ou la revendication 8, dans laquelle le film mince est préparé par une technique choisie parmi la pulvérisation, le dépôt à la tournette, le dépôt-évaporation, le trempage-retrait, la technique de Langmuir-Blodgett, le dépôt électrochimique et la polymérisation *in situ* d'un monomère précurseur du polymère.

10. Utilisation selon l'une quelconque des revendications précédentes, dans laquelle la détection du ou des composés nitrés par le capteur chimique est réalisée par mesure d'une variation de masse du polymère ou de conductivité électrique du composite.

11. Utilisation selon l'une quelconque des revendications 1 à 10, dans laquelle le capteur est un capteur gravimétrique.

12. Utilisation selon la revendication 11, dans laquelle le capteur est un capteur à microbalance à quartz.

13. Utilisation selon l'une quelconque des revendications 1 à 10, dans laquelle le capteur est un capteur résistif.

14. Utilisation selon l'une quelconque des revendications précédentes, dans laquelle le capteur est un multicapteur qui comprend un ou plusieurs capteurs gravimétriques et/ou un ou plusieurs capteurs résistifs, l'un au moins de ces capteurs comprenant un polymère ou un composite tel que précédemment défini.

15. Utilisation selon l'une quelconque des revendications précédentes, dans laquelle le ou les composés nitrés à détecter sont choisis parmi les composés nitroaromatiques, les nitramines, les nitrosamines et les esters nitriques.

16. Utilisation selon l'une quelconque des revendications précédentes, dans laquelle le ou les composés nitrés à détecter sont sous forme solide, liquide ou gazeuse.

17. Utilisation selon l'une quelconque des revendications précédentes, dans laquelle le ou les composés nitrés à détecter sont choisis parmi le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène, la trinitrophénylméthylnitramine et le trinitrophénol.

18. Utilisation selon l'une quelconque des revendications précédentes pour la détection d'explosifs.

**Claims**

1. The use of at least one polymer comprising at least one siloxane repeating unit corresponding to the general formula (I) below:

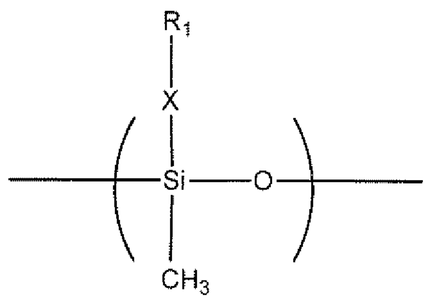

(I)

in which:

X is a saturated or unsaturated, linear hydrocarbon group containing from 1 to 50 carbon atoms, whereas $R_1$ represents a CN group, a group $C(Z)_3$, $CH(Z)_2$ or $CH_2Z$ with Z representing a halogen atom;

or of a composite comprising this polymer and one or more electrically conductive fillers, as sensitive material in a sensor for detecting one or more nitro compounds.

**2.** The use according to claim 1, in which, in formula (I), X represents an alkylene chain containing from 2 to 10 carbon atoms.

**3.** The use according to claim 1 or claim 2, in which the siloxane repeating unit is trifluoropropylmethylsiloxane or cyanopropylmethylsiloxane.

**4.** The use according to any one of the preceding claims, in which the polymer is chosen from polytrifluoropropylmethylsiloxanes and polycyanopropylmethylsiloxanes.

**5.** The use according to claim 4, in which the polymer has an average molecular weight ranging from 50 to 100 000.

**6.** The use according to any one of the preceding claims, in which the conductive filler(s) of the composite is(are) chosen from carbon black particles and metal and metal oxide powders.

**7.** The use according to any one of the preceding claims, in which the polymer or the composite is used in the form of a thin film covering one or both faces of a substrate.

**8.** The use according to claim 7, in which the thin film is from 10 angstroms to 100 microns thick.

**9.** The use according to claim 7 or claim 8, in which the thin film is prepared via a technique chosen from spraying, spin coating, drop coating, dip coating, the Langmuir-Blodgett technique, electroplating and *in situ* polymerization of a precursor monomer of the polymer.

**10.** The use according to any one of the preceding claims, in which the detection of the nitro compound(s) by the chemical sensor is performed by measuring a variation in the mass of the polymer or in the electrical conductivity of the composite.

**11.** The use according to any one of claims 1 to 10, in which the sensor is a gravimetric sensor.

**12.** The use according to claim 11, in which the sensor is a quartz microbalance sensor.

**13.** The use according to any one of claims 1 to 10, in which the sensor is a resistive sensor.

**14.** The use according to any one of the preceding claims, in which the sensor is a microsensor that comprises one or more gravimetric sensors and/or one or more resistive sensors, at least one of these sensors comprising a polymer or a composite as defined above.

**15.** The use according to any one of the preceding claims, in which the nitro compound(s) to be detected is(are) chosen from nitroaromatic compounds, nitroamines, nitrosamines and nitric esters.

**16.** The use according to any one of the preceding claims, in which the nitro compound(s) to be detected is(are) in solid, liquid or gaseous form.

**17.** The use according to any one of the preceding claims, in which the nitro compound(s) to be detected is(are) chosen from nitrobenzene, dinitrobenzene, trinitrobenzene, nitrotoluene, dinitrotoluene, trinitrotoluene, dinitrofluorobenzene, dinitrotrifluoromethoxybenzene, aminodinitrotoluene, dinitrotrifluoromethylbenzene, chlorodinitrotrifluoromethylbenzene, hexanitrostilbene, trinitrophenylmethylnitramine and trinitrophenol.

**18.** The use according to any one of the preceding claims, for detecting explosives.

## Patentansprüche

**1.** Verwendung von wenigstens einem Polymer, umfassend wenigstens ein Siloxan-Wiederholungsmuster, der folgenden allgemeinen Formel (I) entsprechend:

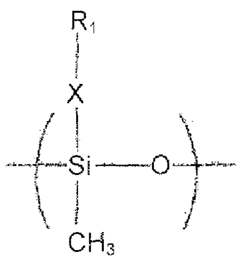

(I)

in der X eine lineare, gesättigte oder ungesättigte Kohlenstoffgruppe ist und 1 bis 50 Kohlenstoffatome umfasst, während $R_1$ eine CN-Gruppe, eine $C(Z)_3$-, $CH(Z)_2$- oder $CH_2Z$-Gruppe repräsentiert, wobei Z ein Halogenatom repräsentiert;
oder von einem dieses Polymer und eine oder mehrere elektrisch leitfähige Beimengungen enthaltenden Verbundstoff als empfindliches Material in einem Sensor, bestimmt zur Detektion von einer oder mehreren Nitroverbindungen.

**2.** Verwendung nach Anspruch 1, bei der X in der Formel (I) eine Alkylenkette mit 2 bis 10 Kohlenstoffatomen repräsentiert.

**3.** Verwendung nach Anspruch 1 oder Anspruch 2, bei der das Siloxan-Wiederholungsmuster das Trifluorpropylmethylsiloxan oder das Cyanopropylmethylsiloxan ist.

**4.** Verwendung nach einem der vorhergehenden Ansprüche, bei der das Polymer ausgewählt wird unter den Polytrifluorpropylmethylsiloxanen und den Polycyanopropylmethylsiloxanen.

**5.** Verwendung nach Anspruch 4, bei der das Polymer ein von 50 bis 100 000 gehendes mittleres Molekulargewicht hat.

**6.** Verwendung nach einem der vorhergehenden Ansprüche, bei der die leitfähige(n) Beimengung(en) des Verbundstoffs ausgewählt wird (werden) unter Russpartikeln und Metall- oder Metalloxidpulvern.

**7.** Verwendung nach einem der vorhergehenden Ansprüche, bei der das Polymer oder der Verbundstoff in Form eines Dünnfilms bzw. einer Dünnschicht, eine oder beide Seiten eines Substrats bedeckend, verwendet wird.

**8.** Verwendung nach Anspruch 7, bei der der Dünnfilm eine Dicke von 10 Ångström bis 100 Mikrometer hat.

**9.** Verwendung nach Anspruch 7 oder Anspruch 8, bei der der Dünnfilm mittels einer Technik hergestellt wird, die ausgewählt wird unter Sputtern, Schleudern, Dip-Coating, der Langmuir-Blodgett-Technik, der elektrochemischen

Abscheidung und der Insitu-Polymerisation eines Vorläufermonomers des Polymers.

**10.** Verwendung nach einem der vorhergehenden Ansprüche, bei der die Detektion der Nitroverbindung(en) durch den chemischen Sensor durch Messen einer Veränderung der Masse des Polymers oder der elektrischen Leitfähigkeit des Verbundstoffs realisiert wird.

**11.** Verwendung nach einem der Ansprüche 1 bis 10, bei der der Sensor ein gravimetrischer Sensor ist.

**12.** Verwendung nach Anspruch 11, bei der der Sensor ein Quarz-Mikrowaagensensor ist.

**13.** Verwendung nach einem der Ansprüche 1 bis 10, bei der der Sensor ein resistiver Sensor ist.

**14.** Verwendung nach einem der vorhergehenden Ansprüche, bei der der Sensor ein Multisensor ist, der einen oder mehrere gravimetrische Sensoren und/oder einen oder mehrere resisitve Sensoren umfasst, wobei wenigstens einer dieser Sensoren ein Polymer oder einen Verbundstoff wie oben definiert umfasst.

**15.** Verwendung nach einem der vorhergehenden Ansprüche, bei der die zu detektierende(n) Nitroverbindung(en) ausgewählt wird (werden) unter den nitroaramatischen Verbindungen, den Nitraminen, den Nitrosaminen und den Nitroestern.

**16.** Verwendung nach einem der vorhergehenden Ansprüche, bei der die zu detektierende(n) Nitroverbindung(en) im festen, flüssigen oder gasförmigen Zustand ist (sind).

**17.** Verwendung nach einem der vorhergehenden Ansprüche, bei der die zu detektierende(n) Nitroverbindung(en) ausgewählt wird (werden) unter Nitrobenzyl, Dinitrobenzol, Trinitrobenzol, Nitrotoluol, Dinitrotoluol, Trinitrotoluol, Dinitrofluorbenzol, Dinitrofluormethoxybenzol, Aminodinitrotoluol, Dinitrotrifluormethoxybenzol, Chlordinitrotrifluormethylbenzol, Nexanitrostilben, Trinitrophenylmethylnitramin und Trinitrophenol.

**18.** Verwendung nach einem der vorhergehenden Ansprüche zur Detektion von Sprengstoffen.

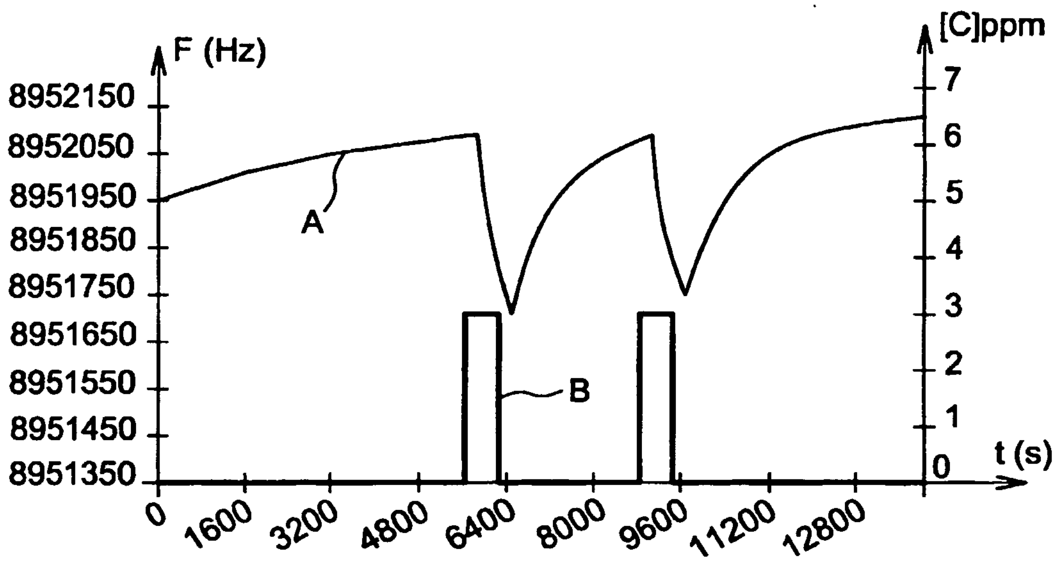
F (Hz)
[C]ppm
8952150
8952050
8951950
8951850
8951750
8951650
8951550
8951450
8951350
7
6
5
4
3
2
1
0
A
B
t (s)
0
1600
3200
4800
6400
8000
9600
11200
12800

FIG. 1

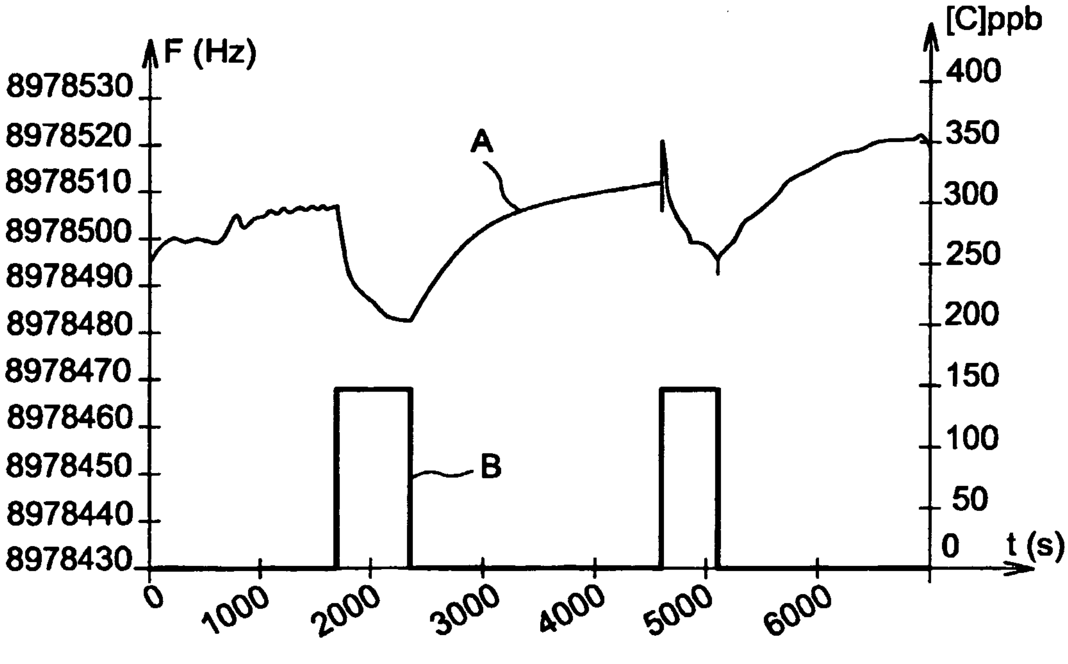
F (Hz)
[C]ppb
8978530
8978520
8978510
8978500
8978490
8978480
8978470
8978460
8978450
8978440
8978430
400
350
300
250
200
150
100
50
0
A
B
t (s)
0
1000
2000
3000
4000
5000
6000

FIG. 2

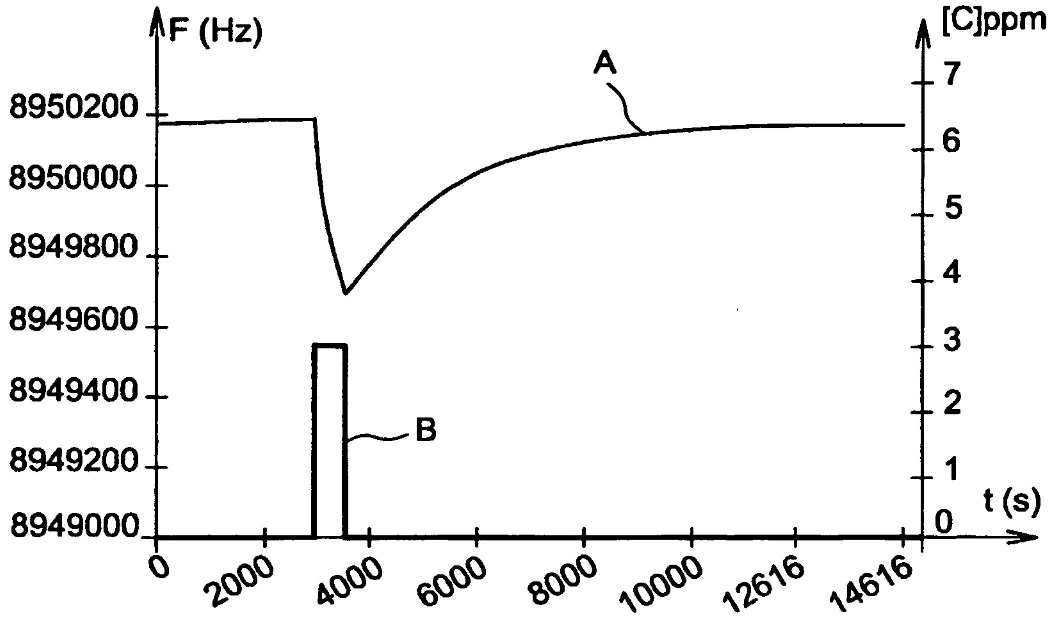
F (Hz)
[C]ppm
A
B
8950200
8950000
8949800
8949600
8949400
8949200
8949000
7
6
5
4
3
2
1
0
t (s)
0
2000
4000
6000
8000
10000
12616
14616

FIG. 3

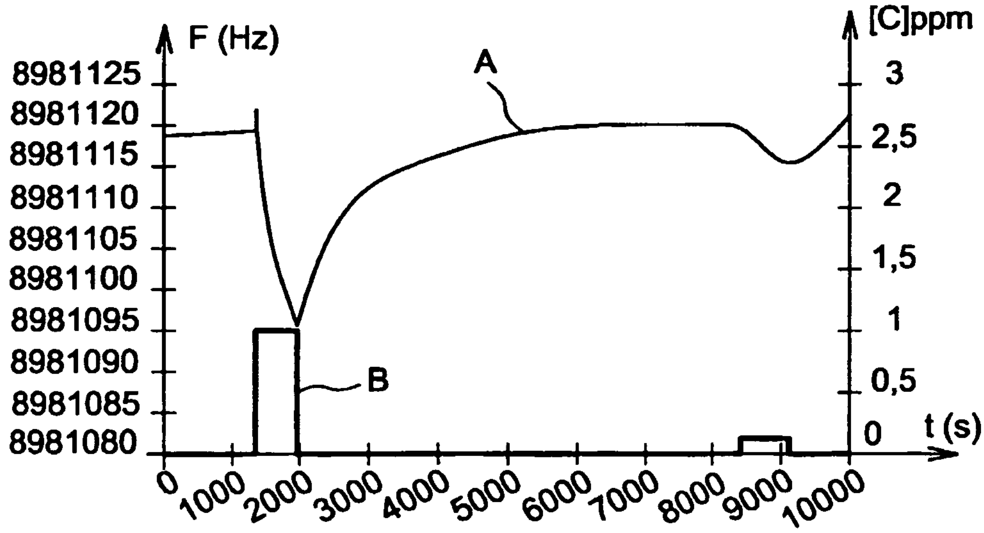
F (Hz)
[C]ppm
A
B
8981125
8981120
8981115
8981110
8981105
8981100
8981095
8981090
8981085
8981080
3
2,5
2
1,5
1
0,5
0
t (s)
0
1000
2000
3000
4000
5000
6000
7000
8000
9000
10000

FIG. 4

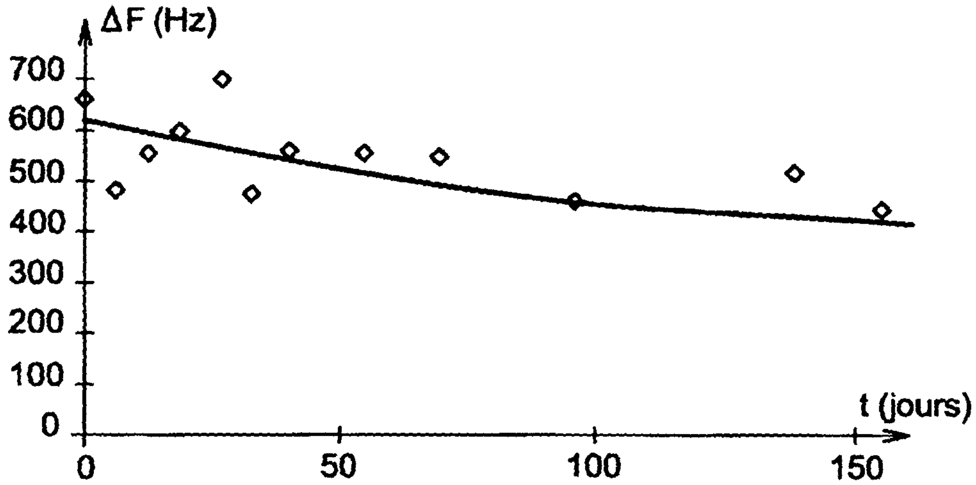


FIG. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **Content et al.** *Chem. Eur. J.,* 2000, vol. 6, 2205
- **Sanchez-Pedrono et al.** *Anal. Chim. Acta,* vol. 182, 285
- **Yang et al.** *Langmuir,* 1998, vol. 14, 1505
- **Nelli et al.** *Sens. Actuators B,* 1993, vol. 13-14, 302
- **Yang et al.** *J. Am. Chem. Soc.,* 1998, vol. 120, 11864
- **McGill et al.** *Sensors and Actuators B65,* 2000, 5-9